# FASCICULE DE BREVET EUROPEEN

(11) **EP 0 767 162 B1**
(45) Date de publication et mention de la délivrance du brevet: **08.12.1999**
(21) Numéro de dépôt: 96401929.3
(22) Date de dépôt: 10.09.1996
(51) Int. Cl.: C07C 59/52, C07C 51/41

(54) **Complexe orthohydroxymandélate de sodium-phénol-eau, procédé de préparation et utilisation pour isolement de l'orthohydroxymandélate de sodium**
Natrium-Orthohydroxymandelat-Phenol-Wasser-Komplexe, Verfahren zu deren Herstellung sowie Verwendung zur Isolation des Natrium Orthohydroxymandelats
Sodium orthohydroxylate-phenol-water complexes, process for preparing same and application for isolation of sodium orthohydroxymandelate

(30) Priorité: 04.10.1995 FR 9511658
(43) Date de publication de la demande: 09.04.1997
(73) Titulaire: Clariant (France) S.A., 92800 Puteaux (FR)
(72) Inventeur: Sidot, Christian, 60200 Compiegne (FR); Tarlier, Michel, 60410 Verberie (FR)
(74) Mandataire: Rinuy, Santarelli

(56) Documents cités:
- Aucun document pertinent relevé

## Description

L'invention a pour objet la préparation d'un complexe cristallisé orthohydroxymandélate de sodium-phénol-eau, son procédé de préparation sous forme cristallisée et l'utilisation de ce complexe pour préparer l'orthohydroxymandélate de sodium, soit sous forme cristallisée, soit sous forme d'une solution avec un degré de pureté élevé.

La préparation de l'acide orthohydromandélique (AOHM) ou de son sel de sodium (OHMNa) sous forme suffisamment pure et facilement manipulable n'a pu être réalisée industriellement.

Howe R., Rao B.S et Heynecker H. (J. Chem. Soc. (1967), 2510 - 2514) ont obtenu l'acide orthohydroxymandélique sous forme d'une gomme visqueuse à partir de la réduction de l'acide 2-hydroxy-α-oxobenzèneacétique par l'hydrogène dans une solution aqueuse d'hydrogénocarbonate de sodium en présence de catalyseur d'Adams préréduit.

Selon cet article, plusieurs tentatives pour isoler l'isomère ortho de l'acide orthohydroxymandélique ont échoué. Par exemple, il a été proposé de traiter l'aldéhyde salicylique par le cyanure d'hydrogène et hydrolyser le nitrile résultant à l'aide d'une solution concentrée d'acide chlorhydrique. Il a aussi été proposé de réduire l'acide 2-hydroxy-α-oxobenzèneacétique par l'amalgame au sodium. Il a enfin été proposé de diazoter le sel de sodium de l'acide 2-aminomandélique et de chauffer le composé diazonium résultant avec de l'acide sulfurique dilué.

Mais, dans tous les cas, les produits de réaction ont toujours été obtenus sous forme visqueuse avec un faible rendement et selon des procédés difficiles à mettre en oeuvre.

Actuellement, les procédés connus pour obtenir l'acide orthohydroxymandélique ou ses sels alcalins partent de la réaction de l'acide glyoxylique sur le phénol.

Hoefnagel A.J. Peters J.A., Van Bekkum H. (Recl. Trav. Chim. Pays-Bas (1988), 107 (3), 242-7) ont montré que la condensation de l'acide glyoxylique avec du phénol pouvait être catalysée par certains ions métalliques et qu'en opérant en milieu aqueux dilué, à pH 5, à 100°C, en présence de cations métalliques trivalents tels que l'aluminium, le chrome, le fer; il était possible d'obtenir des sélectivités élevées en position ortho. Toutefois, en raison d'une part, d'une dilution importante qui fournit une faible productivité et d'autre part, de l'obtention d'un taux élevé en produits disubstitués qui conduit à des mélanges complexes dont il est difficile ensuite d'isoler l'acide orthohydroxymandélique recherché, ce procédé ne peut pas être exploité à l'échelle industrielle.

Schouteeten A. et Christidis Y. ont récemment décrit dans FR-A-2.687.143 une méthode permettant d'obtenir l'acide orthohydroxymandélique avec un rendement de 86,5% par condensation de l'acide glyoxylique sur le phénol en présence d'une amine tertiaire, de préférence la tributylamine et de quantités catalytiques de cations métalliques trivalents, de préférence Al₂(SO₄)₃, les produits secondaires de cette réaction étant l'acide parahydroxymandélique et un produit disubstitué en quantité très faible.

Mais dans les deux cas cités ci-dessus, il reste à isoler l'acide orthohydroxymandélique ou ses sels alcalins à l'état pur.

Récemment Hoefnagel A.J. Peters J.A., Van Bekkum H ont décrit dans WO-94.14746 une méthode de séparation des isomères ortho et para des sels alcalins de l'acide orthohydroxymandélique par extraction sélective des mélanges aqueux. En faisant une extraction du mélange avec un solvant organique aprotique polaire, par exemple l'acétone, la méthylcétone, le tétrahydrofuranne, l'acétate d'éthyle, il a été trouvé que le sel de sodium de l'isomère ortho était très soluble dans ces solvants. Mais un tel procédé exige de nombreuses extractions et évaporations et l'utilisation répétée de solvants, ce qui rend le procédé difficilement industrialisable, coûteux et source de pollution. De plus, aucun des produits obtenus n'a la pureté exigée dans certaines applications comme par exemple la synthèse de principes actifs pour l'industrie pharmaceutique.

De façon inattendue, la demanderesse a découvert qu'en partant d'un mélange orthohydroxymandélate de sodium (OHMNa) - parahydroxymandélate de sodium (PHMNa) tel que décrit par exemple dans FR-A-2.687.143, et en introduisant dans ce milieu du phénol, on obtenait un complexe OHMNa-phénol-eau, sous forme de cristaux.

Ce complexe cristallisé OHMNa-phénol-eau permet d'obtenir ultérieurement soit de l'OHMNa hydraté et cristallisé de grande pureté, soit une solution aqueuse d'OHMNa de grande pureté. Ceci est particulièrement important pour une telle molécule qui peut servir d'intermédiaire de synthèse en chimie fine.

De façon plus particulière, l'invention concerne un nouveau complexe orthohydroxymandélate de sodium-phénol-eau de formule (I) 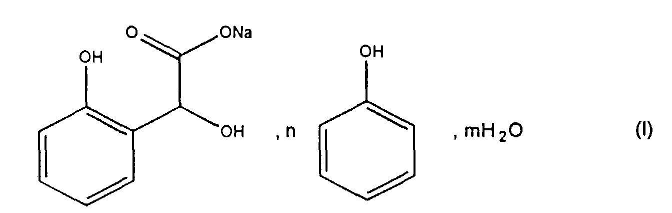 dans lequel n est compris entre 0,8 et 1,2, de préférence entre 0,9 et 1,1 et dans lequel m est compris entre 0,8 et 1,2. Le terme complexe est entendu comme un composé d'addition, c'est à dire un composé dont la molécule résulte de la juxtaposition de deux ou de plusieurs autres. La particularité du complexe obtenu est qu'il a pu être isolé sous forme cristallisée.

La présente invention concerne également un procédé de préparation du complexe cristallisé ci-dessus décrit consistant à introduire du phénol (P₂) dans une solution aqueuse S à un pH compris entre 7 et 9 pour obtenir le complexe attendu que l'on cristallise. La solution aqueuse S est une solution telle qu'issue de la réaction de phénol (P₁) sur l'acide glyoxylique en présence d'une amine tertiaire et d'un cation trivalent, puis traitement à l'aide d'une solution d'un hydroxyde alcalin, notamment comme décrit dans FR-A-2.687.143.

De façon préférentielle, la solution aqueuse S est issue d'une telle réaction, et préparée dans les conditions suivantes :

Le rapport molaire phénol (P₁) sur acide glyoxylique est supérieur à 1, de préférence supérieur à 4.

Le rapport molaire amine tertiaire sur acide glyoxylique est compris entre 0,8 et 1,2, de préférence entre 0,9 et 1,1.

Le cation trivalent est choisi dans le groupe constitué par le cation trivalent de l'aluminium, du chrome, du fer, et de préférence de l'aluminium.

Le rapport molaire cation métallique sur acide glyoxylique est compris entre 0,001 et 0,1, et de façon préférentielle est égal à environ 0,05.

La concentration pondérale de la solution aqueuse d'acide glyoxylique utilisée est de 50 % environ.

La température de réaction est supérieure ou égale à 50°C, de préférence inférieure à 100°C.

Cette solution S comportera en majorité de l'orthohydroxymandélate de sodium (OHMNa), du parahydroxymandélate de sodium (PHMNa) et éventuellement de faibles quantités d'acides hydroxybenzenediglycoliques substitués en -2,4 et en -2,6 (AHBDG).

Pour former le complexe cristallisé désiré, le rapport molaire entre l'OHMNa contenu dans S et le phénol (P₂) que l'on introduit dans S, sera notamment d'une valeur comprise entre 0,5 et 1,5, préférentiellement entre 0,9 et 1,1. Le pH de la réaction sera compris entre 7 et 9.

La cristallisation du complexe (I) sera effectuée avantageusement entre 30 et 60°C, préférentiellement entre 40° et 50°C. Après filtration et séchage des cristaux, on obtient le complexe (I) attendu.

On a pu démontrer qu'il s'agissait bien d'un complexe, notamment par son spectre infrarouge qui est différent du résultat de l'addition des spectres individuels de l'OHMNa et du phénol en mélange. On a également pu démontrer par analyse thermogravimétrique que l'eau du complexe était bien de l'eau constitutive du complexe.

La présente invention a également pour objet l'application du complexe cristallisé précité pour préparer l'orthohydroxymandélate de sodium (OHMNa) de formule (II) : 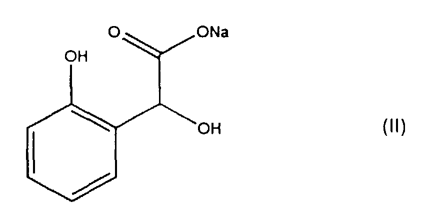 ainsi qu'un procédé de préparation de l'orthohydroxymandélate de sodium (OHMNa) de formule (II) à partir dudit complexe.

Le procédé est caractérisé par le fait que l'on traite le complexe ci-dessus à l'aide d'un solvant qui ne solubilise pas l'OHMNa mais qui solubilise le phénol. On utilisera plus particulièrement une cétone comme la méthylisobutylcétone (MIBC) ou l'acétone ; préférentiellement on utilisera la MIBC. Le produit obtenu après séchage est de l'OHMNa quasiment anhydre sous forme d'une poudre.

La présente invention concerne encore un procédé de préparation de l'orthohydroxymandélate de sodium (OHMNa) en solution aqueuse à partir du complexe précité.

Le procédé consiste à traiter le complexe (I) par un mélange eau-solvant organique S', S' étant non miscible à l'eau et solubilisant le phénol. S' pourra être une cétone comme le MIBC ou un éther comme le tertioamylméthyléther (TAME) le méthyltertiobutyléther (MTBE), le diisopropyléther (DIPE). On utilisera préférentiellement le TAME. Dans les conditions décrites ci-dessus on a obtenu de l'OHMNa de très grande pureté, en particulier sans trace de phénol.

Les exemples suivants sont donnés à titre indicatif pour mieux comprendre l'invention.

### EXEMPLE 1 :

On ajoute 95 g de phénol (0,91 mole) en solution aqueuse à 90 % à un mélange de 1000 g de solution aqueuse obtenue selon le mode opératoire décrit dans l'exemple 2 de FR-A-2.687.143 et contenant essentiellement :
- 173 g (0,91 mole) d'orthohydroxymandélate de sodium (OHMNa)
- 34,2 g (0,18 mole) de parahydroxymandélate de sodium (PHMNa)
- 5,7 g (0,02 mole) de sels de sodium d'acides hydroxybenzènediglycoliques substitués en -2,4 et en -2,6 (AHBDGNa)
dont le pH a été ajusté à 7. On chauffe à 45°C sous agitation pendant 30 minutes, puis on refroidit lentement sous faible agitation . La cristallisation s'amorce vers 40°C.

On laisse revenir à température ambiante pendant 12 heures puis on refroidit avec un bain de glace à 5°C.

On obtient alors ainsi un précipité qui est filtré puis lavé par 250 ml d'eau bipermutée à 5°C. On récupère ainsi 244 g de produit humide qui, après séchage à l'étuve sous pression réduite de 50 mm de mercure à 50°C, donne 195g d'un produit solide blanc crème à orange dont le point de fusion par la méthode au tube capillaire est de 105°C. Le rendement obtenu est de 71%.

Le dosage du produit final par chromatographie liquide à haute performance (HPLC) indique la présence d'un complexe ayant la composition molaire 1mole OHMNa - 0,92 mole phénol - 1,15 mole eau. Cette analyse est confirmée par le dosage du complexe effectué par acidimétrie.

Le spectre infrarouge du complexe trouvé est différent du spectre résultant de l'addition des spectres de l'OHMNa et du phénol, ce qui prouve que l'on est bien en présence d'un complexe et non pas de la juxtaposition de trois entités chimiques.

L'analyse thermogravimétrique du complexe indique que l'eau présente est de l'eau constitutive du complexe.

### EXEMPLE 2

887 g du complexe OHMNa-phénol-eau isolé, préparé selon l'exemple 1 sont mis en suspension dans 3000 cm³ de méthylisobutylcétone (MIBC) et laissés 12 H sous agitation lente à température ambiante. Il se forme un précipité qui est lavé par deux fois 1000 cm³ de MIBC.

On récupère ainsi 575 g de produit humide qui est séché à l'étuve à 80°C sous pression réduite pendant 12 H, et qui conduit à 536 g de produit séché, soit un rendement de 96 % par rapport au produit attendu.

L'analyse thermogravimétrique (ATG) et le dosage d'eau par la méthode de Karl Fischer indiquent que le produit est quasiment anhydre. L'ATG permet de dire que le produit se dégrade à partir de 150°C sans fusion détectée. L'alcalimétrie et l'acidimétrie indiquent un produit à 99 % de pureté.

Les spectres RMN et IR sont conformes à la structure attendue du produit, c'est à dire celle de l'OHMNa.

### EXEMPLE 3

2600 g du complexe humide isolé préparés selon l'exemple 1 sont mis en suspension dans un réacteur de 10 litres dans 4300 g d'eau bi-permutée à température ambiante. On ajoute alors trois litres de tertioamylméthyléther (TAME) et on agite 1 heure à 25°C. Tout le précipité est solubilisé. Après arrêt de l'agitation, on obtient deux phases distinctes. Après séparation de ces phases et lavage de la phase aqueuse par trois fois 1000 cm³ de TAME et élimination du TAME par concentration au rotavapor, on obtient 5300 g d'une phase aqueuse dont analyse par HPLC indique une concentration en OHMNa de 22,4 % en masse et l'absence de phénol. Le rendement de la réaction est de 99% et le produit a une pureté de 100%.

## Revendications

1. Un complexe orthohydroxymandélate de sodium-phénol-eau cristallisé de formule (I) 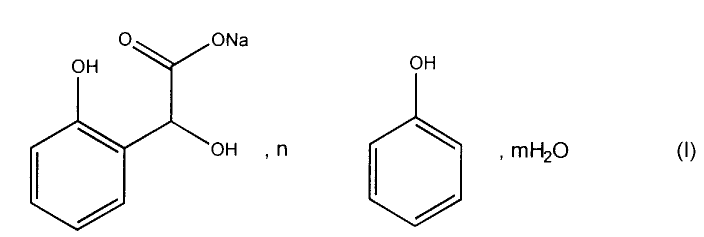 dans laquelle n est compris entre 0,8 et 1,2 et m est compris entre 0,8 et 1,2.

2. Procédé de préparation du complexe tel que défini à la revendication 1, caractérisé en ce que l'on introduit du phénol (P₂) dans une solution aqueuse S à un pH compris entre 7 et 9 pour obtenir le produit attendu que l'on cristallise, étant entendu que
- la solution aqueuse S est telle qu'issue de la réaction de phénol (P₁) sur l'acide glyoxylique en présence d'une amine tertiaire et d'un cation trivalent, puis traitement à l'aide d'une solution d'un hydroxyde alcalin, et
- le rapport molaire de l'OHMNa contenu dans la solution aqueuse S au phénol (P₂) que l'on introduit dans S est compris entre 0,5 et 1,5.

3. Procédé selon la revendication 2, caractérisé par le fait que la solution aqueuse S est préparée dans des conditions telles que :
- le rapport molaire phénol (P₁) sur acide glyoxylique est supérieur à 1,
- le rapport molaire amine tertiaire sur acide glyoxylique est compris entre 0,8 et 1,2,
- le cation trivalent est un cation de l'aluminium, du chrome ou du fer.
- le rapport molaire cation métallique sur acide glyoxylique est compris entre 0,001 et 0,1,
- l'acide glyoxylique est utilisé en solution aqueuse de concentration pondérale de 50 % environ.
- la température de réaction est supérieure ou égale à 50°C.

4. Procédé selon l'une des revendications 2 ou 3, caractérisé par le fait que la solution aqueuse S comprend de l'orthohydroxymandélate de sodium (OHMNa), du parahydroxymandélate de sodium (PHMNa) et éventuellement des acides hydroxybenzenediglycoliques substitués en -2,4 et en -2,6 (AHBDG).

5. Procédé selon la revendication 4, caractérisé par le fait que le rapport molaire de l'OHMNa contenu dans la solution aqueuse S sur le phénol (P₂) entrant en réaction est compris entre 0,9 et 1,1.

6. Application du complexe tel que défini à la revendications 1 à la préparation de l'orthohydroxymandélate de sodium (OHMNa) de formule (II)

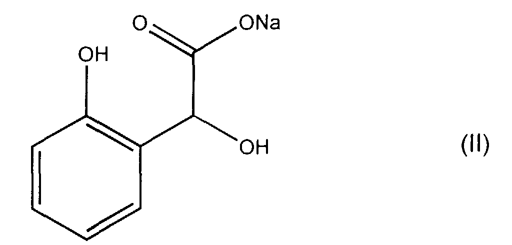

7. Procédé de préparation de l'orthohydroxymandélate de sodium (OHMNa) de formule (II) à partir du complexe tel que défini à la revendication 1, caractérisé par le fait que l'on traite ledit complexe à l'aide d'un solvant solubilisant le phénol mais ne solubilisant pas l'OHMNa.

8. Procédé selon la revendication 7, caractérisé par le fait que le solvant est une cétone telle que la méthylisobutylcétone (MIBC), ou l'acétone.

9. Procédé de préparation de l'OHMNa de formule (II) en solution aqueuse à partir du complexe tel que défini à la revendication 1, caractérisé en ce que l'on traite ledit complexe à l'aide d'un mélange eau/solvant organique S', S' étant non miscible à l'eau et solubilisant le phénol.

10. Procédé selon la revendication 9, caractérisé par le fait que S' est le di-isopropyléther, le méthyltertiobutyléther, la méthylisobutylcétone, le tertioamylméthyléther.

## Patentansprüche

1. Kristallisierter Natriumorthohydroxymandelat-Phenol-Wasser-Komplex der Formel I 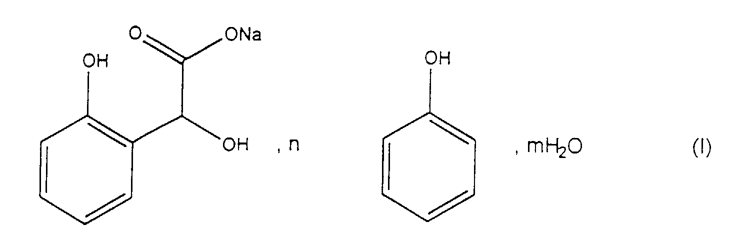 in welcher n einen Wert zwischen 0,8 und 1,2 hat und m einen Wert zwischen 0,8 und 1,2 hat.

2. Verfahren zur Herstellung des gemäß Beispiel 1 definierten Komplexes, dadurch gekennzeichnet, daß man Phenol (P₂) in eine wäßrige Lösung S bei einem pH zwischen 7 und 9 einführt, um das erwartete Produkt zu erhalten, das man kristallisiert, wobei
- die wäßrige Lösung S diejenige ist, die aus der Reaktion von Phenol (P₁) mit Glyoxylsäure in Gegenwart einen tertiären Amins und eines dreiwertigen Kations und der anschließenden Behandlung mit einer alkalischen Hydroxidlösung stammt, und
- das molare Verhältnis des in der wäßrigen Lösung S enthaltenen OHMNa zu Phenol P₂, das man in S einführt, zwischen 0,1 und 1,5 liegt.

3. Verfahren nach Anspruch 2, dadurch gekennzeichnet, daß die wäßrige Lösung S unter solchen Bedingungen hergestellt wird, daß
- das molare Verhältnis von Phenol (P₁) zu Glyoxylsäure größer als 1 ist;
- das molare Verhältnis von tertiärem Amin zu Glyoxylsäure zwischen 0,8 und 1,2 liegt;
- das dreiwertige Kation ein Kation von Aluminium, Chrom oder Eisen ist;
- das molare Verhältnis von Metallkation zu Glyoxylsäure zwischen 0,001 und 0,1 liegt;
- die Glyoxylsäure in wäßriger Lösung einer Gewichtskonzentration von etwa 50% verwendet wird;
- die Reaktionstemperatur höher als oder gleich 50°C ist.

4. Verfahren nach einem der Ansprüche 2 oder 3, dadurch gekennzeichnet, daß die wäßrige Lösung S Natriumorthohydroxymandelat (OHMNa), Natriumparahydroxymandelat (PHMNa) und gegebenenfalls 2,4- und 2,6-substituierte Hydroxybenzoldiglykolsäuren (AHBDG) enthält.

5. Verfahren nach Anspruch 4, dadurch gekennzeichnet, daß das molare Verhältnis von in der wäßrigen Lösung S enthaltenem OHMNa zu dem in Reaktion tretenden Phenol (P₂) zwischen 0,9 und 1,1 liegt.

6. Verwendung des in Anspruch 1 definierten Komplexes zur Herstellung von Natriumorthohydroxymandelat (OHMNa) der Formel II:

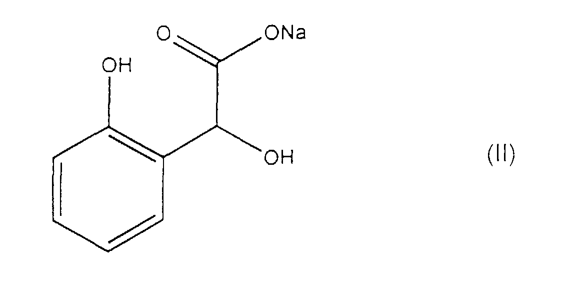

7. Verfahren zur Herstellung von Natriumorthohydroxymandelat (OHMNa) der Formel II aus dem gemäß Anspruch 1 definierten Komplex, dadurch gekennzeichnet, daß man diesen Komplex mit einem Lösungsmittel behandelt, das das Phenol löslich macht, jedoch das OHMNa nicht löslich macht.

8. Verfahren nach Anspruch 7, dadurch gekennzeichnet, daß das Lösungsmittel ein Keton, z.B. Methylisobutylketon (MIBK) oder Aceton, ist.

9. Verfahren zur Herstellung von OHMNa der Formel II in wäßriger Lösung aus dem gemäß Beispiel 1 definierten Komplex, dadurch gekennzeichnet, daß man diesen Komplex mit einer Mischung aus Wasser und organischem Lösungsmittel S' behandelt, wobei S' mit Wasser nicht mischbar ist und das Phenol löst.

10. Verfahren nach Anspruch 9, dadurch gekennzeichnet, daß S' Diisopropylether, Methyl-t-butylether, Methylisobutylketon oder t-Amylmethylether ist.

## Claims

1. A crystallized sodium orthohydroxymandelate-phenol-water complex of formula (I): 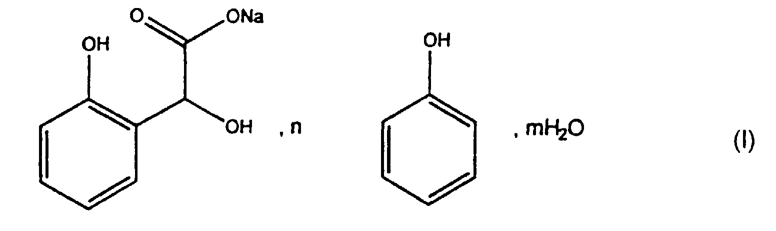 in which n is comprised between 0.8 and 1.2 and m is comprised between 0.8 and 1.2.

2. Preparation process for the complex as defined in claim 1, characterized in that phenol (P₂) is introduced into an aqueous solution S at a pH comprised between 7 and 9 in order to obtain the expected product which is crystallized, it being understood that:
- the aqueous solution S being such as that resulting from the reaction of phenol (P₁) with glyoxylic acid in the presence of a tertiary amine and of a trivalent cation, then treatment using a solution of an alkaline hydroxide, and
- the molar ratio of the NaOHM contained in the aqueous solution S to the phenol (P₂) which is introduced into S is comprised between 0.5 and 1.5.

3. Process according to claim 2, characterized by the fact that the aqueous solution S is prepared under conditions such that:
- the molar ratio of phenol (P₁) to glyoxylic acid is higher than 1,
- the molar ratio of tertiary amine to glyoxylic acid is comprised between 0.8 and 1.2,
- the trivalent cation is a cation of aluminium, chromium or iron,
- the molar ratio of metallic cation to glyoxylic acid is comprised between 0.001 and 0.1,
- the glyoxylic acid is used in aqueous solution with a concentration by weight of about 50%,
- the reaction temperature is higher than or equal to 50°C.

4. Process according to one of claims 2 or 3, characterized by the fact that the aqueous solution S contains sodium orthohydroxymandelate (NaOHM), sodium parahydroxymandelate (NaPHM) and optionally hydroxybenzenediglycolic acids substituted in position -2,4 and in position -2,6 (HBDGA).

5. Process according to claim 4, characterized by the fact that the molar ratio of NaOHM contained in the aqueous solution S to phenol (P₂) entering into the reaction is comprised between 0.9 and 1.1.

6. Use of the complex as defined in claims 1 to for the preparation of sodium orthohydroxymandelate (NaOHM) of formula (II):

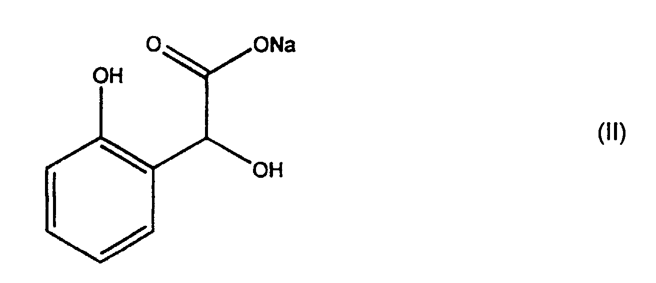

7. Preparation process for sodium orthohydroxymandelate (NaOHM) of formula (II) from the complex as defined in claim 1, characterized by the fact that said complex is treated using a solvent which solubilizes phenol but which does not solubilize NaOHM.

8. Process according to claim 7 characterized by the fact that the solvent is a ketone such as methylisobutylketone (MIBK), or acetone.

9. Preparation process for NaOHM of formula (II) in aqueous solution from the complex as defined in claim 1, characterized in that said complex is treated using a water/organic solvent S' mixture, S' being non-miscible with water and solubilizing phenol.

10. Process according to claim 9, characterized by the fact that S' is diisopropylether, methyltertiobutylether, methylisobutylketone, tertioamylmethylether.
